# EUROPÄISCHE PATENTANMELDUNG

(11) **EP 1 016 654 A2**
(43) Veröffentlichungstag der Anmeldung: **05.07.2000**
(21) Anmeldenummer: 99124309.8
(22) Anmeldetag: 06.12.1999
(51) Int. Cl.: C07C 303/22, C07C 309/44, C07C 309/58

(54) **Verfahren zur Herstellung von substituierten aromatischen und heteroaromatischen Aldehyden und Carbonsäuren**

(30) Priorität: 28.12.1998 AT 217498; 28.04.1999 AT 74899
(71) Anmelder: DSM Fine Chemicals Austria GmbH, 4021 Linz (AT)
(72) Erfinder: Steinbauer, Gerhard, 4470 Enns (AT); Giselbrecht, Karlheinz, 4061 Pasching (AT); Schöftner, Manfred, 4020 Linz (AT); Reiter, Klaus, 4020 Linz (AT)
(74) Vertreter: Klostermann, Ingrid

(57) **Zusammenfassung**

Verfahren zur Herstellung von substituierten aromatischen und heteroaromatischen Aldehyden oder Carbonsäuren der Formel 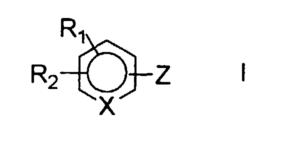 in der R₁ und R₂ H, C₁-C₄ Alkyl, C₁-C₄ Alkoxy, OH, NO₂, CN, COOH, CONH₂, COOR, NH₂, SO₃H oder Halogen und R C₁-C₄ Alkyl bedeuten, wobei R₁ und R₂ nicht gleichzeitig H sind, X C oder N und Z CHO oder COOH bedeuten, aus den entsprechenden symmetrischen Stilbenverbindungen der Formel II 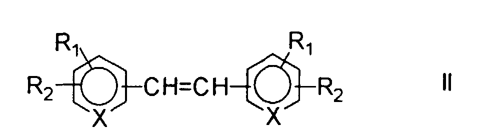 in der in der R₁, R₂ und X wie oben definiert sind durch wässrige Ozonolyse, und anschließender Isolierung eines Aldehyds oder Oxidation zur entsprechenden Carbonsäure.

## Beschreibung

Aromatische und heteroaromatische Aldehyde und Carbonsäuren, wie etwa 4-Nitro-2-sulfobenzaldehyd oder -benzoesäure, sind Zwischenprodukte bei der Herstellung von Herbiziden bzw. Pflanzenwachstumsregulatoren.

In DE 42 36 902 ist die Herstellung von 4-Nitro-2-sulfobenzoesäure über KMnO₄-Oxidation aus 4-Nitrotoluol-2-sulfonsäure beschrieben. Dieser Weg eignet sich jedoch nicht für eine umweltfreundliche, großtechnische Herstellung der gewünschten Verbindung. Dies gilt auch für andere bekannte Oxidationsmethoden, wie etwa mit Natriumhypochlorit und Nickelperoxid. Aus Helv. Chimica Acta 15, 576, 583 (1932) ist die Verwendung von 4,4*'*-Dinitrostilben-2,2*'*-disulfonsäure als Ausgangsprodukt bekannt. Zur Oxidation wird jedoch wiederum KMnO₄ eingesetzt.
Aus diesem Grund wird nach neuen Synthesewegen zur Herstellung von aromatischen und heteroaromatischen Carbonsäuren und Aldehyden gesucht, die die gewünschten Endprodukte auch im großtechnischen Maßstab auf umweltfreundliche Weise ermöglichen.

Gegenstand der vorliegenden Erfindung ist demnach ein Verfahren zur Herstellung von substituierten aromatischen und heteroaromatischen Aldehyden oder Carbonsäuren der Formel

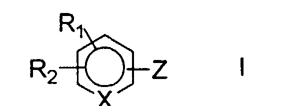

in der R₁ und R₂ H, C₁-C₄ Alkyl, C₁-C₄ Alkoxy, OH, NO₂, CN, COOH, CONH₂, COOR, NH₂, SO₃H oder Halogen und R C₁-C₄ Alkyl bedeuten, wobei R₁ und R₂ nicht gleichzeitig H sind,
X C oder N und Z CHO oder COOH bedeuten, das dadurch gekennzeichnet ist, dass eine entsprechende symmetrische Stilbenverbindung der Formel 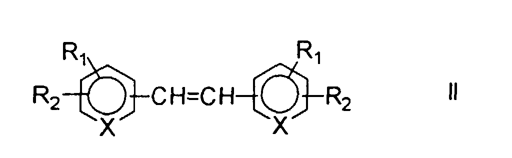 in der R₁, R₂ und X wie oben definiert sind, entweder direkt oder als Salz in Wasser gelöst wird und bei einer Temperatur von 0 bis +100°C mit Ozon umgesetzt wird, worauf ein Gemisch bestehend aus dem entsprechenden Aldehyd der Formel I und Hydroperoxid erhalten wird, aus welchem
a) nach saurer oder alkalischer Zersetzung des Hydroperoxids der entsprechende Aldehyd der Formel I isoliert wird oder das
b) direkt unter saurer oder alkalischer Zersetzung des Hydroperoxids und unter Zugabe eines Oxidationsmittels zur entsprechenden Carbonsäure der Formel I oder deren Salz umgesetzt wird, die bzw. das anschließend isoliert wird.

Das erfindungsgemäße Verfahren eignet sich zur Herstellung von Verbindungen der Formel I.

Diese Verbindungen können aromatische oder heteroaromatische Aldehyde oder Carbonsäuren sein. Der Substituent Z bedeutet daher CHO oder COOH. In o-, m- oder p-Stellung dazu kann ein C-Atom des aromatischen Ringes durch ein N-Atom ersetzt sein, sodass X C oder N bedeutet. Weiters weist der Ring noch die Substituenten R₁ und R₂ auf die ebenfalls in o, m- oder p-Stellung zu Z positioniert sein können. Ist eine dieser Positionen durch X=N ersetzt, so weist das N-Atom keine Substituenten auf.
R₁ und R₂ bedeuten in der Formel I H, C₁-C₄ Alkyl, C₁-C₄ Alkoxy, OH, NO₂, CN, COOH, CONH₂, NH₂, COOR, SO₃H oder Halogen, wie Cl, Br, F. R kann C₁ - C₄ Alkyl bedeuten. R₁ und R₂ bedeuten jedoch nicht gleichzeitig H. Bevorzugt ist R₁ NO₂ und R₂ SO₃H.

Beispiele für durch das erfindungsgemäße Verfahren herstellbare Verbindungen sind demnach 2-Sulfobenzoesäure, 4-Nitro-2-Sulfobenzoesäure, 4-Nitro-2-Sulfobenzaldehyd, 4-Aminobenzoesäure, Therephthalsäure, substituierte Pyridincarbonsäuren, wie etwa 2,3-Pyridindicarbonsäure, u.s.w. oder deren Na- oder K-Salze.
Bevorzugt wird 4-Nitro-2-sulfobenzaldehyd oder -benzoesäure, bzw. das Na- oder K-Salz von 4-Nitro-2-sulfobenzoesäure hergestellt.

Als Ausgangsverbindungen dienen die entsprechenden symmetrischen Stilbenverbindungen der Formel II, in der die Substituenten R₁ und R₂ wie für die Verbindungen der Formel I definiert sind.
Weiters ist, wenn heteroaromatische Endprodukte hergestellt werden, jeweils ein C-Atom der Ringe durch ein N-Atom ersetzt.
Geeignete Verbindungen der Formel II werden direkt oder in Form ihrer Salze eingesetzt.
Als Salze eignen sich beispielsweise Na- oder K-Salze von Säuren oder Chloride, Nitrate, Sulfate, Phosphate von basischen Gruppen.

Beispiele für geeignete Ausgangsverbindungen sind daher Stilbene wie etwa Stilben-2,2'-disulfonsäure, 4,4'-Dinitrostilben-2,2'-disulfonsäure, 4,4'-Vinylendianilin, 4,4'-Vinylendipyridin, 4,4'-Stilbendicarbonsäure, u.s.w. bzw. Salze dieser Verbindungen.

Die Ausgangsverbindungen werden in soviel Wasser gelöst, daß eine 1 bis 50%ige, bevorzugt eine 5 bis 15 %ige Lösung erhalten wird.
Ist die Ausgangsverbindung selbst nicht wasserlöslich, so kann durch Zusatz von geeigneten Basen oder Säuren die wässrige Lösung des entsprechenden Salzes hergestellt werden.

Die so erhaltene Lösung wird solange mit einem ozonführenden O₂-Strom begast, bis die äquivalente Menge an Ozon bzw. ein Überschuß aufgenommen wird.
Das Ende und somit die Dauer der Reaktion ist durch den Verbrauch der theoretischen Ozonmenge gegeben und läßt sich weiters durch einen gleichzeitig auftretenden erhöhten Ozondurchbruch leicht feststellen.
Das Ende der Reaktion kann ferner leicht durch eine geeignete Inprozeßkontrolle auf das Abreagieren der Ausgangsverbindung festgestellt werden.
Die Temperatur der Ozonolyse beträgt 0 bis 100°C. Bevorzugt wird eine Temperatur von +5 bis +80°C, besonders bevorzugt von +15 bis +70°C gewählt.

Nach beendeter Ozonolyse liegt ein Gemisch bestehend aus gleichen molaren Teilen an dem entsprechenden Aldehyd der Formel I und dem entsprechenden Hydroperoxid vor.

Das Hydroperoxid wird sodann sauer oder alkalisch zersetzt. Dementsprechend wird das Reaktionsgemisch nach der Ozonolyse in wässriger saurer Lösung erwärmt oder solange eine wässrige Lösung einer Base zugegeben, bis im Reaktionsgemisch kein Peroxid mehr gefunden wird. Aus dem Hydroperoxid kann nach der Zersetzung entweder die entsprechende Säure oder der Aldehyd entstehen.
Ist der Aldehyd das gewünschte Endprodukt, so wird dieser aus dem Gemisch isoliert. Die Bildung von Aldehyd oder Säure aus dem Hydroperoxid hängt von den Substituenten der Verbindung der Formel I ab.
Bevorzugt wird ein Aldehyd der Formel I isoliert, der als Substituent R₁ NO₂ und als Substituent R₂ H oder SO₃H aufweist.
Zur Isolierung des Aldehyds wird das Hydroperoxid bevorzugt alkalisch zersetzt. Besonders bevorzugt wird dazu Natronlauge verwendet, die bis zum Erreichen eines alkalischen pH-Wertes, bevorzugt über pH 8 zugesetzt wird. Anschließend wird das Gemisch abgekühlt und der Aldehyd auskristallisiert.

Ist die entsprechende Carbonsäure das gewünschte Endprodukt, so wird dem nach der Ozonolyse erhaltenen Reaktionsgemisch, welches Aldehyd und Hydroperoxidverbindung enthält, sofort, ohne Isolierung des Aldehyds ein Oxidationsmittel zugegeben.
Als Oxidationsmittel eignen sich zum Beispiel Wasserstoffperoxid, Persäuren, Ozon, molekularer Sauerstoff, Natriumhypochlorit oder Natriumperborat. Bevorzugt wird Wasserstoffperoxid oder eine Persäure eingesetzt. Das Oxidationsmittel wird mindestens in äquimolarem Verhältnis zu der im Reaktionsgemisch vorhandenen Aldehydmenge, also in mindestens 0,5 molarem Verhältnis zum eingesetzten Stuben verwendet. Die Temperatur der Oxidation ist je nach verwendetem Oxidationsmittel unterschiedlich und liegt in einem Temperaturbereich von 0 bis 100°C.

Die saure oder basische Zersetzung des Hydroperoxids kann dabei sowohl vor als auch nach der Oxidation, aber auch gleichzeitig mit der Oxidation erfolgen. Bevorzugt erfolgt eine saure Zersetzung des Hydroperoxides.
Nach beendeter Oxidation wird die entsprechende Carbonsäure bzw. deren Salz, bevorzugt das Na- oder K-Salz, beispielsweise entweder direkt durch Kristallisation aus dem wässrigen Reaktionsgemisch, beispielsweise durch Einengen, Abkühlen oder durch Zusatz eines geeigneten Fällungsmittels, kristallisiert , oder vorher mit einem organischen Lösungsmittel extrahiert und das Zielprodukt aus dem Extrakt kristallisiert.
Wird als Edukt das Natriumsalz der entsprechenden Verbindung eingesetzt und ist das K-Salz der Carbonsäure das gewünschte Endprodukt, so wird dieses beispielsweise durch Zugabe von Kaliumsalzen, wie Kaliumchlorid, Kalilauge, Kaliumformiat, Kaliumacetat, Kaliumcarbonat u.s.w., ausgefällt.
Die Mutterlauge, die bei der Aufarbeitung des Reaktionsgemisches anfällt, kann nach Einengen wieder bei einem neuen Oxidationsansatz zugegeben werden. Dies ist insbesondere von Vorteil, wenn die Peroxidzersetzung mittels Essigsäure erfolgt, da mit der Mutterlauge auch die Essigsäure recycliert wird.

Durch das erfindungsgemäße Verfahren können die gewünschten Aldehyde und Carbonsäuren, gegebenenfalls in Form ihrer Salze auf umweltfreundlichem Weg erhalten werden, da durch die Verwendung von symmetrischen Stilbenen als Ausgangsmaterial so gut wie kein Abfall anfällt.
Die Zielprodukte können dabei in hoher Reinheit und in hohen Ausbeuten erhalten werden.
Besonders geeignet ist das erfindungsgemäße Verfahren zur Herstellung von 4-Nitro-2-sulfobenzaldehyd oder -benzoesäure bzw. des Natrium- oder Kaliumsalzes dieser Verbindung.

### Beispiel 1:

In eine Lösung von 5,2 g 4,4'-Dinitrostilben-2,2'-disulfonsäure-Dinatriumsalz in 70 ml Wasser wurde bei 30°C mit einer Geschwindigkeit von 100 Liter/Stunde ein Sauerstoffstrom, welcher 50 g/m³ Ozon enthielt, eingeleitet. Nach 8 Minuten war die Aufnahme von Ozon beendet.
Anschließend wurde solange 4N NaOH zugegeben, bis ein konstanter pH-Wert von 9,5 erreicht war. Ein Peroxidtest ergab, daß praktisch kein Peroxid mehr in der Lösung vorhanden war.
Die Lösung wurde auf 5°C gekühlt, das auskristallisierte Produkt wurde abgesaugt und mit wenig 5°C kaltem Wasser gewaschen.
Die Mutterlauge wurde im Vakuum auf ein Volumen von 30 ml eingedampft, nochmals auf 5°C abgekühlt, das kristallisierte Produkt abgesaugt, mit kaltem Wasser gewaschen und mit dem ersten Kristallisat vereinigt. Nach dem Trocknen im Vakuum wurden 4,7 g 4-Nitro-2-sulfobenzaldehyd, Natriumsalz (85 % der Theorie) erhalten. Das Produkt liegt als Monohydrat vor.
Die Struktur wurde mittels ¹H-NMR, ¹³C-NMR, IR und Elementaranalyse bestätigt.
- ¹H-NMR:: δ= 10,9 ppm (1H; -CHO), 8,5 ppm (1H; Ar-H), 8,3 (1H; d, J= 8 Hz, Ar-H), 8,0 (1H; d, J= 8 Hz, Ar-H)
- 13C-NMR:: δ= 194 ppm (-CHO), 153/151/139/131/126/124 (6Ar-C)

Elementaralnalyse: C₇H₆NO₇SNA. H₂O (271,18)
ber. 31,0 % C; 2,2 % H; 5,2 % N 5,0 %; Na 8,5 %, H₂O 6,6 %
gef. 31,2 % C; 2,2 % H; 5,0 % N 5,2 %, Na 8,5 %, H₂O 6,9 %

### Beispiel 2:

In eine Lösung von 300 g (0,414 mol) 4,4'-Dinitrostilben-2,2'-disulfonsäure-Dinatriumsalz in 3750 ml Wasser wurde bei 33°C ein Sauerstoffstrom, welcher 45 g/m³ Ozon enthielt, eingeleitet. Nach 38 Minuten war die Aufnahme von Ozon beendet.
Es wurden 4000 ml Hydroxyperoxid (HPO)-Lösung erhalten, die 0,47 mol Hydroperoxid enthielt.
2000ml der so erhaltenen HPO-Lösung wurden sodann vorgelegt, mit 200 ml Essigsäure versetzt und 23,5ml 30%-iges Wasserstoffperoxid zugetropft. Die Reaktionslösung wurde innerhalb einer Stunde auf 90°C erhitzt, wobei ab 85° C eine leichte Gasentwicklung auftrat. Nach einer Stunde auf 90°C lag der HPO-Gehalt bei 0,35mol. Insgesamt wurde die Reaktionslösung 13h auf 90°C gehalten, wobei dem Reaktionsgemisch innerhalb dieser 13 Stunden eine 2. Portion Wasserstoffperoxid (23,5 ml) und eine Portion Peressigsäure (0,207 mol) zugesetzt wurden. Der Peroxidgehalt sank auf 0,066 mol ab.

Anschließend wurden 1600 ml Wasser bei 60°C Badtemperatur und 70 mbar abdestilliert. Es wurden 600 ml einer klaren Lösung mit 0,04 mol HPO-Gehalt als Rückstand erhalten. Dieser wurde mit 33g Natriumchlorid versetzt, wodurch ein Feststoff ausfiel, der abgesaugt wurde. Es wurden 67,5g (trocken) an Feststoff erhalten, die aus 177g Wasser und 354g Essigsäure umkristallisiert wurden.
Ausbeute 40g 4-Nitro-2-sulfobenzoesäure Natriumsalz

### Beispiel 3:

Analog Beispiel 2 wurde eine HPO-Lösung mit 0,44 mol HPO erhalten, wovon 2100ml (pH 2,9) vorgelegt und mit 1000ml Essigsäure (pH 1,7) versetzt wurden. Anschließend wurden 100g 30%iges Wasserstoffperoxid zugegeben und das Reaktionsgemisch innerhalb einer Stunde auf 80°C erhitzt. Insgesamt wurde die Reaktionslösung 18h auf 80°C gehalten, wobei dem Reaktionsgemisch innerhalb dieser 18 Stunden zwei weitere Portionen Wasserstoffperoxid (0,207 mol bzw. 0,44 mol) zugesetzt wurden. Der Peroxidgehalt sank auf 0,10 mol ab.
Der so erhaltenen Lösung mit pH 1,4 wurde konz. Schwefelsäure (72,7g) bis zum Erreichen von pH 0,2 zugegeben. Anschließend wurden 2000ml bei 60°C Badtemperatur und 90 mbar abdestilliert, worauf die Kristallisation begann. Der Destillationsrückstand wurde auf 15°C gekühlt und der ausgefallene Feststoff abgesaugt.
Ausbeute 99,4g 4-Nitro-2-sulfobenzoesäure Natriumsalz
1107g Mutterlauge wurden mit 75g Natriumchlorid ausgesalzen und der ausgefallene Feststoff abgesaugt, wodurch weitere 108,5g 4-Nitro-2-sulfobenzoesäure Natriumsalz erhalten wurden.
Schmelzpunkt: > 350°C
NMR-Reinheit: > 99%

### Beispiel 4:

Analog Beispiel 2 wurde eine HPO-Lösung mit 0,60 mol HPO erhalten, wovon 1030ml (0,30mol PO; pH 2,6) vorgelegt und mit 200ml Ameisensäure versetzt wurden. Anschließend wurden 66,6g 30%iges Wasserstoffperoxid zugegeben und das Reaktionsgemisch innerhalb einer Stunde auf 80°C erhitzt.
Insgesamt wurde die Reaktionslösung 6h auf 80°C gehalten, wobei dem Reaktionsgemisch innerhalb dieser 6 Stunden zwei weitere Portionen Wasserstoffperoxid (je 66,6g) zugesetzt wurden. Der Peroxidgehalt sank auf 0,052 mol ab.
758ml (801g) wurden bei 60°C Badtemperatur und 90 mbar abdestilliert und der Destillationsrückstand anschließend unter Vakuum auf 10°C gekühlt. Bei 35°C Innentemperatur begann die Kristallisation.
Der Feststoff wurde abgesaugt und getrocknet, wobei 34,9g 4-Nitro-2-sulfobenzoesäure Natriumsalz (21,6% d. Th. bezogen auf eingesetztes Stilben) erhalten wurden.
532g Mutterlauge wurden mit 25g Natriumchlorid ausgesalzen und der ausgefallene Feststoff abgesaugt, wodurch eine Gesamtausbeute von 105g 4-Nitro-2-sulfobenzoesäure Natriumsalz (65% d. Th. bezogen auf eingesetztes Stilben) erhalten wurden.

### Beispiel 5:

Analog Beispiel 2 wurde eine HPO-Lösung mit 0,60 mol HPO erhalten, wovon 2000ml (pH 2,6) vorgelegt und mit 200ml Ameisensäure versetzt wurden. Anschließend wurden 132,2g 30%iges Wasserstoffperoxid zugegeben und das Reaktionsgemisch innerhalb von 45 min auf 80°C erhitzt.
Insgesamt wurde die Reaktionslösung 6h auf 80°C gehalten, wobei dem Reaktionsgemisch innerhalb dieser 6 Stunden eine weitere Portion Wasserstoffperoxid (132,2g) zugesetzt wurden.
Bei 80°C wurden 147,2g (1,5mol) Kaliumacetat zugegeben und innerhalb von 2h auf 5°C gekühlt. auf 10°C gekühlt. Bei 35°C - 40°C Innentemperatur begann die Kristallisation.
Der Feststoff wurde abgesaugt, mit 100ml kaltem Wasser gewaschen und getrocknet, wobei 237g 4-Nitro-2-sulfobenzoesäure Kaliumsalz erhalten wurden.

Der Versuch wurde wiederholt, wobei anstelle von Kaliumacetat, einmal Kalilauge und ein weiteres Mal Kaliumchlorid zum Ausfällen des K-Salzes verwendet wurden.

## Patentansprüche

1. Verfahren zur Herstellung von substituierten aromatischen und heteroaromatischen Aldehyden oder Carbonsäuren der Formel 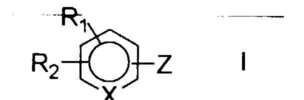 in der R₁ und R₂ H, C₁-C₄ Alkyl, C₁-C₄ Alkoxy, OH, NO₂, CN, COOH, CONH₂, COOR, NH₂, SO₃H oder Halogen und R C₁-C₄ Alkyl bedeuten, wobei R₁ und R₂ nicht gleichzeitig H sind,
X C oder N und Z CHO oder COOH bedeuten, dadurch gekennzeichnet, dass eine entsprechende symmetrische Stilbenverbindung der Formel 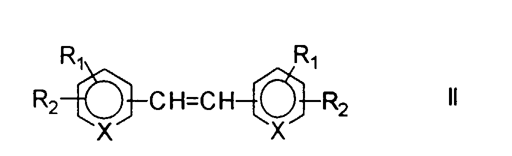 in der R₁, R₂ und X wie oben definiert sind, entweder direkt oder als Salz in Wasser gelöst wird und bei einer Temperatur von 0 bis +100°C mit Ozon umgesetzt wird, worauf ein Gemisch bestehend aus dem entsprechenden Aldehyd der Formel I und Hydroperoxid erhalten wird, aus welchem
a) nach saurer oder alkalischer Zersetzung des Hydroperoxids der entsprechende Aldehyd der Formel I isoliert wird oder das
b) direkt unter saurer oder alkalischer Zersetzung des Hydroperoxids und Zugabe eines Oxidationsmittels zur entsprechenden Carbonsäure der Formel I oder deren Salz umgesetzt wird, die bzw. das anschließend isoliert wird.

2. Verfahren nach Anspruch 1, dadurch gekennzeichnet, dass sich in den Verbindungen der Formel I X, R₁ und R₂ in o-, m- oder p-Stellung zum Substituenten Z befinden können, mit der Maßgabe, daß falls X=N ist, das N-Atom nicht substituiert ist.

3. Verfahren nach Anspruch 2, dadurch gekennzeichnet, dass als Verbindungen der Formel I 2-Sulfobenzoesäure, 4-Nitro-2-sulfobenzoesäure, 4-Nitro-2-sulfobenzaldehyd, 4-Aminobenzoesäure, Therephthalsäure oder substituierte 4-Pyridincarbonsäure oder deren Natrium- oder Kaliumsalze hergestellt werden.

4. Verfahren nach Anspruch 1, dadurch gekennzeichnet, dass die Verbindungen der Formel II direkt, in Form ihrer Alkalisalze von Säuren oder in Form eines Chlorides, Nitrates, Sulfates oder Phosphates von basischen Gruppen in Wasser gelöst werden, sodass eine 1 bis 50%ige Lösung erhalten wird.

5. Verfahren nach Anspruch 1, dadurch gekennzeichnet, dass zur Isolierung des Aldehydes das Gemisch auf einen alkalischen pH- Wert gestellt, das Hydroperoxid zersetzt und der Aldehyd nach Abkühlung auskristallisiert wird.

6. Verfahren nach Anspruch 5, dadurch gekennzeichnet, dass als Aldehyde der Formel I, Aldehyde mit NO₂ und/oder SO₃H als Substituenten isoliert werden.

7. Verfahren nach Anspruch 1, dadurch gekennzeichnet, dass zur Isolierung der Carbonsäuren das Gemisch mit Wasserstoffperoxid, einer Persäure, Ozon, molekularem Sauerstoff, Natriumhypochlorit oder Natriumperborat als Oxidationsmittel in mindestens äquimolaren Verhältnis zu der im Gemisch vorhandenen Aldehydmenge versetzt, das Hydroperoxid entweder vor, nach oder während der Oxidation sauer oder alkalisch zersetzt und die Carbonsäure oder deren Salz durch Extraktion oder Kristallisation isoliert wird.

8. Verfahren nach Anspruch 7, dadurch gekennzeichnet, dass die Zersetzung des Hydroperoxides durch Zugabe einer Säure erfolgt.

9. Verfahren nach Anspruch 7, dadurch gekennzeichnet, dass die Carbonsäure als K-Salz durch Zugabe von Kaliumchlorid, Kalilauge, Kaliumformiat, Kaliumacetat oder Kaliumcarbonat auskristallisiert wird.
